(19) Europäisches Patentamt
European Patent Office
Office européen des brevets

(11) **EP 2 435 861 B1**

(12) **FASCICULE DE BREVET EUROPEEN**

(45) Date de publication et mention de la délivrance du brevet:
**09.09.2020 Bulletin 2020/37**

(21) Numéro de dépôt: **10715907.1**

(22) Date de dépôt: **06.05.2010**

(51) Int Cl.:
*G01V 3/10* (2006.01)    *A61B 5/06* (2006.01)
*G01V 3/08* (2006.01)    *G01R 29/08* (2006.01)
*G01S 13/00* (2006.01)

(86) Numéro de dépôt international:
**PCT/EP2010/056211**

(87) Numéro de publication internationale:
**WO 2010/136316 (02.12.2010 Gazette 2010/48)**

(54) **PROCÉDÉ DE DÉTECTION ET DÉTECTEUR DE PERTURBATEUR, PROCÉDÉ ET SYSTÈME DE LOCALISATION UTILISANT CE PROCÉDÉ**

DETEKTIONSVERFAHREN UND DETEKTOR FÜR EINE UNTERBRECHUNG, POSITIONSBESTIMMUNGSVERFAHREN UND SYSTEM, DASS DAS VERFAHREN VERWENDET

DETECTION METHOD AND DETECTOR OF A DISRUPTION, POSITIONING METHOD AND SYSTEM USING SAID METHOD

(84) Etats contractants désignés:
**AL AT BE BG CH CY CZ DE DK EE ES FI FR GB GR HR HU IE IS IT LI LT LU LV MC MK MT NL NO PL PT RO SE SI SK SM TR**

(30) Priorité:  **26.05.2009   PCT/FR2009/953462**

(43) Date de publication de la demande:
**04.04.2012   Bulletin 2012/14**

(73) Titulaire: **Commissariat à l'Énergie Atomique et aux Énergies Alternatives
75015 Paris (FR)**

(72) Inventeurs:
• **BILLERES, Malvina
  38000 Grenoble (FR)**
• **CATTIN, Viviane
  38120 Saint Egrève (FR)**

(74) Mandataire: **Colombo, Michel et al
Brevinnov
310 avenue Berthelot
69008 Lyon (FR)**

(56) Documents cités:
EP-A1- 0 993 804      EP-A2- 0 319 637
EP-A2- 2 052 681      WO-A1-96/41119
US-A1- 2002 093 338   US-B1- 6 172 499

**Description**

L'invention concerne un procédé de détection et un détecteur de perturbateur d'amplitude de champ magnétique à proximité d'une source de ce champ magnétique. L'invention concerne également un procédé et un système de localisation d'un objet dans un référentiel utilisant ce procédé de détection.

Un perturbateur d'amplitude de champ magnétique est ici défini comme étant tout objet qui altère ou déforme l'amplitude d'un champ magnétique émis à proximité. Par exemple, le perturbateur peut être une pièce conductrice. Dans ce cas, l'altération de l'amplitude du champ magnétique peut être due à l'apparition de courant de Foucault (« Eddy current » en anglais) dans la pièce conductrice. La pièce conductrice est par exemple une pièce métallique. Le perturbateur peut également être une pièce magnétique telle qu'une pièce paramagnétique ou ferromagnétique. Dans le cas des pièces ferromagnétiques, l'altération de l'amplitude du champ magnétique est due au fait que cette pièce déforme les lignes du champ magnétique.

Par exemple, les procédés connus de détection de perturbateurs sont utilisés au sein de procédés de localisation d'un objet dans un référentiel à l'aide d'au moins une paire de mesures. Chaque paire de mesures comporte :

- une source monoaxe apte à émettre des champs magnétiques à plusieurs fréquences, et
- un capteur apte à mesurer l'amplitude des champs magnétiques émis par cette source monoaxe,
- l'un de la source monoaxe et du capteur étant solidaire du référentiel et l'autre de la source monoaxe et du capteur étant solidaire de l'objet.

Une source monoaxe de champ magnétique est une source qui émet préférentiellement le champ magnétique le long d'un seul axe. Par exemple, une bobine dont les spires sont enroulées autour d'un même axe est une source monoaxe de champ magnétique et l'axe d'émission préférentielle est confondu avec l'axe d'enroulement des spires.

Dans la suite de cette description, on désigne par le terme « amplitude » du champ magnétique, l'amplitude A du champ en phase ou l'amplitude Ch du module du champ qui est fonction à la fois du champ en phase et du champ en quadrature de phase. Ces amplitudes A et Ch sont proportionnelles.

L'amplitude A est donnée par la relation suivante :

$$A = \sqrt{Ix^2 + Iy^2 + Iz^2}$$

$$(1)$$

où :

- Ix, Iy et Iz sont les composantes en phase du champ, mesurées selon trois directions orthogonales entre elles X, Y et Z.

L'amplitude Ch est donnée par les relations suivantes :

$$Ch = \sqrt{Ch_x^2 + Ch_y^2 + Ch_z^2} \qquad (2)$$

$$Ch_x = 2\sqrt{(Ix^2 + Qx^2)} \qquad (3)$$

$$Ch_y = 2\sqrt{(Iy^2 + Qy^2)} \qquad (4)$$

$$Ch_z = 2\sqrt{(Iz^2 + Qz^2)} \qquad (5)$$

où :
- Qx, Qy, Qz sont les composantes en quadrature de phase du champ magnétique, mesurées selon les trois directions orthogonales X, Y et Z.

Typiquement, le capteur apte à mesurer l'amplitude du champ magnétique telle que définie ci-dessus est un capteur tri-axe capable de mesurer les composantes en phase et/ou en quadrature de phase selon les axes X, Y et Z. Pour localiser l'objet, ces procédés comportent :

- l'émission d'un champ magnétique à une fréquence donnée par la source monoaxe et la mesure de ce champ magnétique par le capteur de la même paire,
- la localisation de l'objet dans le référentiel à partir des mesures réalisées par la ou les paire(s) de mesure.

De tels procédés de localisation sont souvent utilisés en médecine pour localiser une sonde ou un cathéter à l'intérieur d'un corps humain. Pour de telles applications, la fiabilité de la localisation de la sonde est très importante. Or, dans le milieu médical, il existe de nombreux perturbateurs susceptibles de fausser la localisation. Par exemple, le perturbateur peut être une table d'opération, le scalpel d'un chirurgien, l'armature métallique d'un autre appareil placé à proximité du patient,...etc.

Plusieurs méthodes ont déjà été proposées pour détecter tels perturbateurs (voir par exemple EP 1 502 544 ou EP 0993 804 ou US 2002/092 338 A ou EP 0 319 637 ou EP 2 052 681). Ces méthodes font appel à des calculs complexes. De plus, dans la méthode décrite dans EP 0 993 804, une calibration, préalable à toute détection d'un perturbateur, doit être réalisée.

L'invention vise à remédier à ce problème en détectant simplement la présence d'un perturbateur d'amplitude du champ magnétique émis.

Elle a donc pour objet un procédé de détection d'un perturbateur d'amplitude de champ magnétique conforme à la revendication 1.

Le procédé ci-dessus utilise le fait qu'un perturbateur ne perturbe pas de la même façon l'amplitude du champ magnétique à toutes les fréquences. Typiquement, un perturbateur perturbe l'amplitude du champ magnétique de façon plus ou moins forte selon la fréquence considérée.

Le procédé ci-dessus utilise également la propriété des champs magnétiques selon laquelle, en absence de perturbateur, l'amplitude du champ magnétique mesurée par le capteur varie en fonction de la distance R qui le sépare de la source monoaxe de ce champ magnétique. Cette propriété est indépendante de la fréquence du champ magnétique émis. Plus précisément, l'amplitude du champ magnétique mesurée décroît proportionnellement à $1/R^3$.

Pour la suite des explications, on note $A_i$ et $A_j$ les amplitudes de deux champs magnétiques émis par une même source monoaxe, respectivement, aux fréquences $f_i$ et $f_j$. Nous notons également $Am_i$ et $Am_j$ les amplitudes mesurées par le capteur aux fréquences $f_i$ et $f_j$. Le capteur se trouve à une distance R de la source monoaxe.

En absence de perturbateur, le rapport $Am_i/Am_j$ doit être sensiblement égal au rapport prédéterminé $A_i/A_j$. Ainsi, si l'écart entre ces rapports dépassent le seuil prédéterminé, cela signale la présence d'un perturbateur d'amplitude du champ magnétique. De plus, ceci reste vrai quelle que soit la distance R. Il n'est donc pas nécessaire de connaître cette distance R pour détecter un perturbateur.

Un tel procédé ne nécessite pas de calcul complexe puisqu'il suffit simplement de comparer des rapports d'amplitudes mesurées à un seuil prédéterminé.

Ce procédé permet également de détecter un perturbateur sans recours à une calibration préalable contrairement aux procédés connus.

Enfin, l'utilisation d'une même source monoaxe pour émettre les champs magnétiques de fréquences différentes permet d'augmenter la précision du procédé car il n'est pas nécessaire de corriger des écarts dus à l'utilisation de plusieurs sources monoaxes spatialement espacées les unes des autres pour émettre chacun des champs magnétiques de fréquences différentes.

Les modes de réalisation de ce procédé de détection peuvent comporter une ou plusieurs des caractéristiques des revendications dépendantes.

Ces modes de réalisation du procédé de détection présentent en outre les avantages suivants :

- émettre des champs magnétiques dont les rapports d'amplitudes sont égaux permet de détecter un perturbateur en comparant simplement les rapports d'amplitudes mesurées entre eux sans qu'il soit nécessaire de connaître d'autres informations sur le rapport prédéterminé ;
- mesurer et utiliser uniquement l'amplitude en phase des champs magnétiques simplifie la mise en oeuvre du procédé car il n'est alors pas nécessaire de mesurer l'amplitude de ces mêmes champs magnétiques en quadrature de phase ;
- émettre simultanément les champs magnétiques de fréquences différentes permet d'accélérer la détection d'un perturbateur.

Un mode de réalisation à également pour objet un procédé de localisation d'un objet dans un référentiel à l'aide d'au moins un détecteur conforme à la revendication 4.

Le procédé de localisation ci-dessus présente une précision améliorée puisque les mesures réalisées par les paires de mesures perturbées sont pondérées pour limiter leur impact sur la localisation de l'objet. De plus, c'est la même source monoaxe et le même capteur qui sont utilisés à la fois pour la détection d'un perturbateur et pour la localisation de l'objet.

Les modes de réalisation du procédé de localisation peuvent comporter les caractéristiques de la revendication 5.

Ce mode de réalisation présente en outre l'avantage de permettre la détermination de la position et de l'orientation de l'objet dans un référentiel orthogonal à trois dimensions.

L'invention a également pour objet un support d'enregistrement d'informations comportant des instructions pour la mise en oeuvre des procédés ci-dessus, lorsque ces instructions sont exécutées par un calculateur

L'invention a également pour objet un détecteur d'un perturbateur d'amplitude de champ magnétique conforme à la revendication 7.

Enfin, l'invention à également pour objet un système de localisation d'un objet dans un référentiel conforme à la revendication 9.

L'invention a également pour objet un détecteur d'un perturbateur caractéristique de la revendication 9.

L'invention sera mieux comprise à la lecture de la description qui va suivre, donnée uniquement à titre d'exemple non limitatif et faite en se référant aux dessins sur lesquels :

- la figure 1 est une illustration schématique d'un système de localisation d'un objet dans un référentiel comportant un détecteur de perturbateur ;
- la figure 2 est un organigramme d'un procédé de localisation à l'aide du système de la figure 1 ;
- la figure 3 est un graphe illustrant la mise en oeuvre du procédé de la figure 2 ;
- la figure 4 est un organigramme d'un autre mode de réalisation d'un procédé de localisation à l'aide du système de la figure 1 ; et
- la figure 5 est un graphe illustrant la mise en oeuvre du procédé de la figure 4.

Dans ces figures, les mêmes références sont utilisées pour désigner les mêmes éléments.

Dans la suite de cette description, les caractéristiques et fonctions bien connues de l'homme du métier ne sont pas décrites en détail.

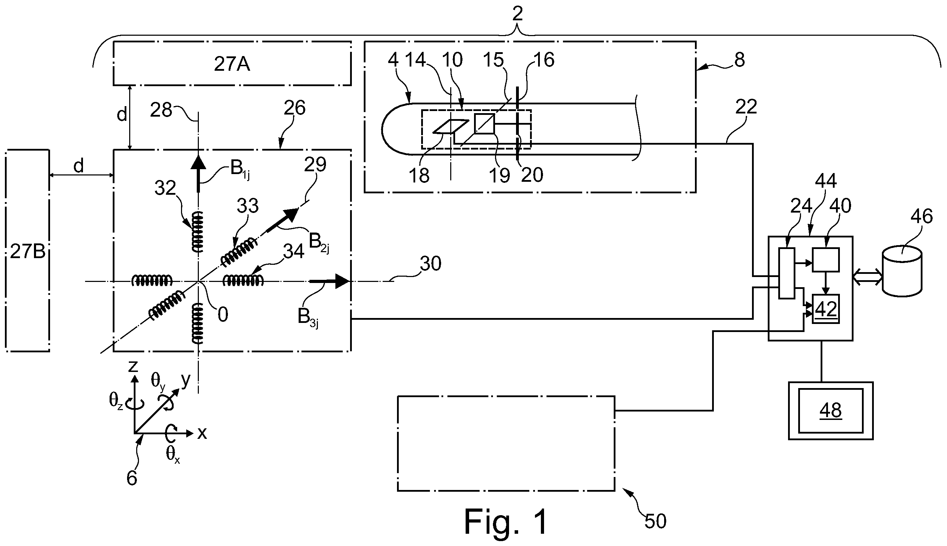

La figure 1 représente un système 2 de localisation d'un objet 4 dans un référentiel 6. L'objet 4 est par exemple une sonde ou un cathéter introduit dans un corps humain 8. L'objet 4 est mobile dans le référentiel 6.

Le référentiel 6 est un référentiel fixe présentant trois axes orthogonaux X, Y et Z.

La localisation de l'objet 4 dans le référentiel 6 consiste ici à trouver sa position x, y, z et son orientation $\theta_x$, $\theta_y$, $\theta_z$. Les angles $\theta_x$, $\theta_y$ et $\theta_z$ représentent l'orientation de l'objet 4, respectivement, par rapport aux axes X, Y et Z.

Pour localiser l'objet 4 dans le référentiel 6, celui-ci est, par exemple, équipé d'un capteur 10 de champs magnétiques.

Le capteur 10 est un capteur tri-axe, c'est-à-dire apte à mesurer le champ magnétique reçu selon trois axes non-colinéaire 14 à 16. Ici ces axes 14 à 16 de mesure sont orthogonaux entre eux. Ces axes sont solidaires de l'objet 4. À cet effet, le capteur 10 incorpore trois transducteurs monoaxe 18 à 20. Chacun de ces transducteurs présente une direction de mesure le long de laquelle sa sensibilité au champ magnétique est maximale. Ici, les directions de mesure des transducteurs 18, 19 et 20 sont confondues, respectivement, avec les axes 14, 15 et 16.

Par exemple, les transducteurs 18, 19 et 20 sont des bobines enroulées, respectivement, autour des axes 14, 15 et 16.

Chacun de ces transducteurs est raccordé par l'intermédiaire d'une liaison filaire souple 22 à une unité 24 de traitement.

L'unité 24 est également raccordée à troix sources tri-axe 26, 27A et 27B de champs magnétiques de manière à avoir une redondance géométrique. Ces sources 26, 27A et 27B sont espacées l'une de l'autre par une distance d. Par exemple, les sources 26, 27A et 27B sont identiques et seule la source 26 est décrite plus en détails.

La source 26 est fixe dans le référentiel 6. Cette source 26 est apte à émettre des champs magnétiques le long de trois axes orthogonaux 28 à 30. Ici, les axes 28 à 30 sont, respectivement, parallèles aux axes Z, Y et X du référentiel 6.

À cet effet, la source 26 est ici composée de trois sources monoaxes 32 à 34. Les sources monoaxes 32 à 34 émettent, respectivement, des champs $B_{1j}$, $B_{2j}$ et $B_{3j}$ le long des directions 28, 29 et 30. L'indice i est un identifiant de la source monoaxe et l'indice j est un identifiant de la fréquence $f_j$ du champ magnétique émis par cette source monoaxe.

Chacune de ces sources monoaxes 32 à 34 peut être modélisée par une source ponctuelle de champs magnétiques. De préférence, les sources monoaxes 32 à 34 sont agencées de manière à ce que leurs sources ponctuelles respectives occupent exactement la même position dans le référentiel 6. Ici cette position est repérée par un point O. Le point O est à l'intersection des axes 28 à 30.

Par exemple, chaque source monoaxe 32 à 34 est constituée d'une seule bobine enroulée autour, respectivement, des axes 28 à 30. Ici, chacune de ces bobines est divisée en deux groupes identiques de spires réparties de façon symétrique de part et d'autre du point O le long de l'axe d'enroulement. Chaque groupe de spires est bobiné dans le même sens le long de l'axe d'enroulement.

La distance la plus courte d qui sépare la source tri-axe 26 des sources tri-axes 27A et 27B est au moins deux et, de préférence au moins trois, fois supérieure à la plus grande dimension de la source 26. La plus grande dimension de la source 26 est par exemple ici la plus grande longueur d'une des sources monoaxes 32 à 34.

L'association d'une seule source monoaxe et d'un seul capteur tri-axe travaillant à la même fréquence de travail forme une paire de mesures. Par exemple, la source monoaxe 32 et le capteur 10 forment une première paire de mesures lorsqu'ils travaillent à la fréquence $f_{1t}$ et une deuxième paire de mesures lorsqu'ils travaillent à la fréquence $f_{2t}$. Ce même capteur 10 associé à la source monoaxe 33 forme une troisième et une quatrième paires de mesures lorsqu'ils travaillent, respectivement, aux fréquences $f_{1t}$ et $f_{2t}$. Dans le mode de réalisation décrit ici chaque source monoaxe travaille aux deux fréquences $f_{1t}$ et $f_{2t}$, il existe donc dix-huit paires de mesures.

L'unité 24 de traitement alimente la source 26 en courant alternatif pour générer les champs magnétiques $B_{ij}$ et acquière les champs magnétiques mesurés par les transducteurs 18 à 20. Dans la suite de cette description, on suppose que le perturbateur est une pièce magnétique non conductrice. Typiquement, pour chaque paire de mesures, l'unité 24 établit l'amplitude $Am_{ij}$ de la composante I du champ magnétique en phase avec le champ magnétique émis.

À cet effet, l'unité 24 est un détecteur synchrone. Un exemple d'un tel détecteur synchrone est décrit en regard de la figure 16 du brevet U.S. 6 528 989. Ainsi, l'unité 24 ne sera pas décrite ici plus en détail. Toutefois, l'unité 24 est simplifiée par rapport aux détecteurs synchrones connus puisqu'il n'est pas nécessaire d'établir les composantes en quadrature de phase Qx, Qy et Qz.

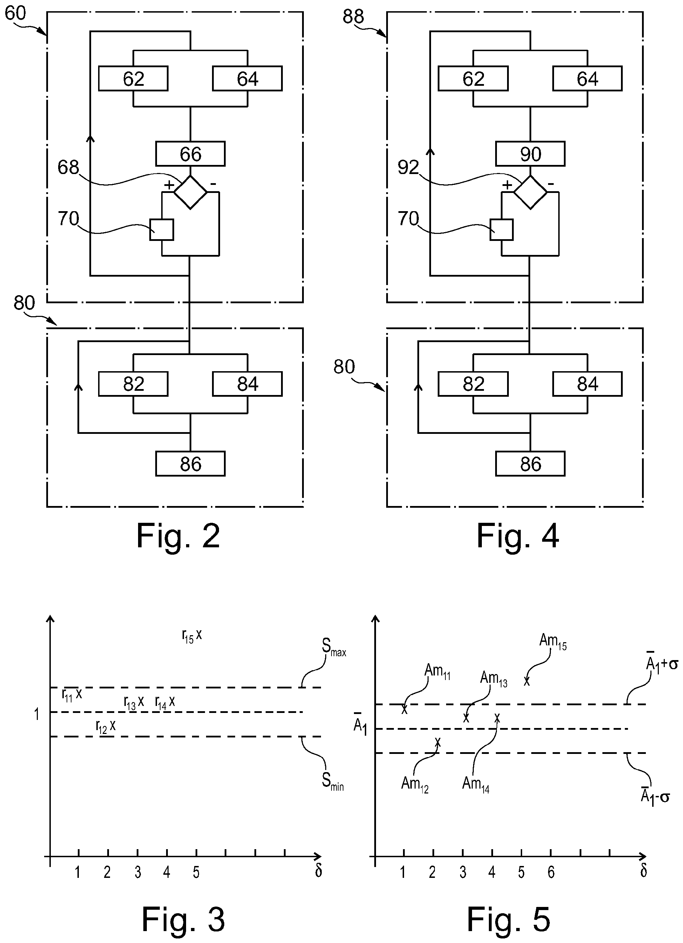

L'unité 24 est raccordée à un indicateur 40 propre à signaler la présence d'un perturbateur magnétique à partir de l'amplitude de la composante I des champs magnétiques mesurés. Le fonctionnement de cet indicateur est décrit plus en détail en regard de la figure 2.

L'association du capteur tri-axe 10, des sources monoaxes, de l'unité 24 et de l'indicateur 40 forme un détecteur de perturbateur de champ magnétique.

L'unité 24 est également raccordée à un module 42 de localisation de l'objet 4 dans le référentiel 6. Typiquement, le module 42 détermine la position et l'orientation de l'objet 4 en résolvant un système d'équations. Ce système d'équations est obtenu en modélisant les interactions magnétiques entre les sources monoaxes et les transducteurs sans tenir compte de la présence de perturbateurs. Dans ce système d'équations, la position x, y et z et l'orientation $\theta_x$, $\theta_y$ et $\theta_z$ de l'objet 4 sont les inconnues alors que les valeurs des autres paramètres sont obtenues à partir des mesures réalisées par le capteur 10. Plus d'informations sur de tels systèmes d'équations peuvent, par exemple, être trouvées dans la demande de brevet EP 1 502 544.

Ici, de préférence, ce système d'équations s'écrit sous la forme d'un filtre de Kalman.

Par exemple, l'unité 24 se présente sous la forme d'une carte électronique tandis que l'indicateur 40 et le module 42 se présentent sous la forme de modules logiciels. A cet effet, le système 2 comprend un calculateur électronique programmable 44 incorporant l'unité 24 et apte à exécuter des instructions enregistrées sur un support d'enregistrement d'informations. Le calculateur 44 est raccordé à une mémoire 46 contenant les instructions pour exécuter le procédé de la figure 2 ou 4 lorsqu'elles sont exécutées par le calculateur 44. Le calculateur 44 est également raccordé à une interface homme-machine 48. Par exemple l'interface homme-machine comprend un écran sur lequel est représenté la position de l'objet 4 dans le référentiel 6.

Le système 2 peut comprendre également d'autres appareils de mesure de grandeurs physiques, autre que des champs magnétiques, représentatives de la position de l'objet 4 dans le référentiel 6. Par exemple, le système 2 comprend un appareil 50 tel qu'un appareil de radiographie ou une caméra. Les mesures de l'appareil 50 sont insensibles à la présence ou l'absence d'un perturbateur magnétique.

Le fonctionnement du système 2 va maintenant être décrit plus en détail en regard du procédé de la figure 2.

Avant la localisation de l'objet 4 dans le référentiel 6, on procède à une phase 60 de détection de perturbateurs. Au début de cette phase, lors d'une étape 62, la source monoaxe 32 émet des champs $B_{11}$, $B_{12}$,... $B_{1j}$ de même amplitude $A_{1j}$ mais, respectivement, à des fréquences $f_1$, $f_2$,... $f_j$ différentes. Ainsi, le rapport entre les amplitudes des champs magnétiques émis est constant et égal à un. Par exemple, les fréquences $f_j$ sont choisies dans une plage allant du 10 Hz à 100 kHz. De préférence, elles sont régulièrement réparties dans la plage choisie. Ces champs $B_{1j}$ peuvent être émis simultanément ou séquentiellement, c'est-à-dire les uns après les autres.

En parallèle, lors d'une étape 64, l'amplitude $Am_{1j}$ des champs magnétiques $B_{1j}$ est mesurée à l'aide du capteur 10 et de l'unité 24.

Ensuite, l'indicateur 40 vérifie si les rapports entre les amplitudes $A_{1j}$ pour différentes fréquences sont sensiblement égaux aux rapports entre les amplitudes $Am_{1j}$ pour ces mêmes fréquences. Etant donné qu'ici les rapports entre les amplitudes $A_{1j}$ sont tous égaux à un, cette vérification revient à vérifier que les amplitudes mesurées $Am_{1j}$ aux différentes fréquences sont sensiblement égales quelle que soit la fréquence.

De nombreuses méthodes sont possibles pour faire cette vérification. Par exemple, ici, l'indicateur 40 calcule lors d'une étape 66, un rapport $r_{1j}$ à l'aide de la formule suivante :

$$r_{1j} = Am_{1j}/Am_{11}$$

Ce rapport $r_{1j}$ est calculé pour toutes les fréquences $f_j$ des champs magnétiques émis par la source monoaxe 32.

Ensuite, lors d'une étape 68, chaque rapport $r_{1j}$ est comparé à un seuil minimum $S_{min}$ et à un seuil maximum $S_{max}$. Ces seuils $S_{min}$ et $S_{max}$ sont situés de part et d'autre de la valeur théorique du rapport $r_{1j}$ en absence de perturbateur (voir figure 3). Dans cette exemple, la valeur théorique est un. Ici, les seuils $S_{min}$ et $S_{max}$ sont choisis de manière à permettre une tolérance autour de cette valeur théorique inférieure à 10 %. Par exemple, une tolérance d'au moins 3% est prévue ici. Par exemple, les seuils $S_{min}$ et $S_{max}$ sont, respectivement, inférieur à 0,95 et supérieur à 1,05.

Si l'un des rapports $r_{1j}$ n'est pas compris entre les seuils $S_{min}$ et $S_{max}$, alors on procède à une étape 70 de signalement de la présence d'un perturbateur d'amplitude. Lors de l'étape 70, cette information est présentée à un être humain par l'intermédiaire de l'interface homme-machine 48. Dans le cas contraire, aucun perturbateur n'est signalé.

Les étapes 62 à 70 sont réitérées pour chaque paire de mesures. Ces itérations peuvent être réalisées séquentiellement, c'est-à-dire les unes après les autres ou en parallèle, c'est-à-dire en même temps.

Une fois que la phase de détection 60 a été réalisée pour chaque paire de mesures, on procède à une phase 80 de localisation de l'objet 4. Au début de cette phase, lors d'une étape 82, chaque source monoaxe émet deux champs magnétiques $B_{im}$. De préférence, les champs magnétiques $B_{im}$ sont émis, respectivement, à des fréquences de travail $f_{1t}$ et $f_{2t}$ choisies parmi les fréquences $f_j$ utilisées lors de l'étape 62.

En parallèle, lors d'une étape 84, ces champs magnétiques $B_{Im}$ sont mesurés à l'aide du capteur 10 et de l'unité 24.

Les étapes 82 à 84 peuvent être réalisées pour une paire de mesures l'une après l'autre ou pour l'ensemble des paires de mesures simultanément.

Une fois les mesures réalisées, celles-ci sont fournies au module 42 qui procède à une étape 86 de détermination de la localisation de l'objet dans le référentiel 6. Lors de cette étape 86, les différentes valeurs mesurées sont utilisées pour paramétrer le système d'équations qui doit ensuite être résolu. Etant donné que l'on dispose de dix-huit paires de mesures, et qu'il faut seulement neuf paires de mesures pour résoudre ce système d'équations, on dispose de nombreuses informations redondantes. Cette redondance des informations est mise à profit par le module 42 en pondérant les mesures perturbées, c'est-à-dire les mesures obtenues par une paire de mesures signalée comme étant perturbée lors de l'étape 70, à l'aide d'un coefficient qui limite l'impact de cette mesure perturbée sur la localisation de l'objet 4. Plus précisément, ce coefficient diminue l'importance des mesures perturbées par rapport aux autres mesures qui n'ont pas été signalées comme perturbées. Ce coefficient de pondération peut être non-nul. Il peut également prendre la valeur zéro ce qui revient à ne tout simplement pas tenir compte des mesures perturbées lors de la détermination de la localisation de l'objet 4.

La figure 4 représente une variante du procédé de la figure 2. Cette variante est identique au procédé de la figure 2 à l'exception que la phase 60 de détection d'un perturbateur est remplacée par une phase 88.

La phase 88 se distingue de la phase 60 par le fait que l'on détecte d'une manière différente que les amplitudes $A_{1j}$ sont sensiblement constantes quelle que soit la fréquence $f_j$. A cet effet, les étapes 66 et 68 sont remplacées, respectivement, par des étapes 90 et 92.

Lors de l'étape 90, on calcule la moyenne $\overline{A}_1$ des amplitudes $Am_{1j}$ mesurées. On calcule également lors de cette étape l'écart-type $\sigma$ par rapport à cette moyenne. L'amplitude $Am_{1j}$ est l'amplitude de la composante I en phase du champ magnétique. Ensuite, lors d'une étape 92, on vérifie que chaque amplitude $Am_{1j}$ est comprise entre des seuils définis en fonction de $\overline{A}_1$ et de l'écart type $\sigma$. Par exemple, ces seuils sont $\overline{A}_1 - \sigma$ et $\overline{A}_1 + \sigma$. Si l'une des amplitudes $Am_{1j}$ mesurées n'est pas comprise entre les seuils $\overline{A}_1 - \sigma$, $\overline{A}_1 + \sigma$, alors on procède à l'étape 70. Dans le cas contraire, aucun perturbateur n'est signalé. Cette façon de vérifier que les amplitudes $Am_j$ sont sensiblement constantes quelle que soit la fréquence $f_j$ est représentée sur la figure 5. Sur cette figure 5, les croix représentent les amplitudes $Am_{1j}$ mesurées.

Ce graphe montre en particulier le signalement d'un perturbateur puisque l'amplitude $Am_{15}$ est au-dessus du seuil $\overline{A}_1 + \sigma$.

Les étapes 90 et 92 constituent une autre façon de vérifier que les rapports entre les amplitudes mesurées pour différentes fréquences sont sensiblement constants quelle que soit la fréquence en absence de perturbateur.

De nombreuses autres modes de réalisation sont possibles. Par exemple, il est possible d'utiliser l'amplitude Ch du module du champ à la place de l'amplitude de la composante I que le perturbateur soit une pièce conductrice ou magnétique. Dans ce cas les composantes I et Q doivent être mesurées. Par contre, seule l'amplitude de la composante I peut être utilisée si le perturbateur est une pièce magnétique non-conductrice.

La localisation et la détection de perturbateurs ont été décrites ici dans le cas particulier où c'est l'objet 4 qui est mobile par rapport à un référentiel 6 fixe. Ce qui a été décrit précédemment s'applique également à la situation inverse où c'est l'objet 4 qui est fixe et le référentiel 6 qui est mobile. Dans ce cas-là, l'objet 4 est fixe et ce sont les sources monoaxes qui se déplacent par rapport à l'objet 4.

Il est également possible d'inverser la position des sources et des capteurs. Par exemple, une ou plusieurs sources tri-axes de champ magnétique sont intégrées dans l'objet et un ou plusieurs capteurs tri-axes sont fixés sans aucun degré de liberté au référentiel 6.

Ce qui a été décrit précédemment s'applique également à des systèmes de localisation de l'objet 4 dans un référentiel à une ou deux dimensions. De même, le système 2 peut être simplifié si l'on ne souhaite pas mesurer la position ou l'orientation de l'objet 4. Dans ces cas-là, le nombre de paires de mesures peut être réduit.

Les champs magnétiques $B_{ij}$ peuvent être multiplexés temporellement ou fréquentiellement. Ainsi, il est possible de mener en parallèle, en cas de multiplexage fréquentiel, plusieurs itérations des étapes 62 à 70 pour différents champs de fréquences différentes.

Il n'est pas nécessaire que les champs magnétiques émis par une bobine soient tous de même amplitude quelle que soit la fréquence. En effet, si l'amplitude du champ magnétique $B_{ij}$ varie en fonction de sa fréquence, il faut alors connaître le rapport entre les amplitudes des champs magnétiques émis par cette bobine à différentes fréquences pour vérifier que les rapports des amplitudes mesurées restent proches des rapports attendus. Par exemple, le rapport attendu entre les amplitudes mesurées des champs magnétiques pour différentes fréquences est déterminé à partir de l'intensité du courant d'alimentation des bobines formant les sources monoaxes 32 à 34. Ces rapports attendus peuvent également être déterminés lors d'une phase de calibration en absence de perturbateur.

Les fréquences $f_j$ utilisées lors de la phase 60 de détection d'un perturbateur sont, en variante, choisies dans des bandes de fréquences libres de parasites électromagnétiques. Par exemple, le dispositif identifie les bandes libres de parasites électromagnétiques en écoutant les champs magnétiques déjà présents dans l'environnement avant de débuter la détection d'un perturbateur.

Lorsqu'un perturbateur est détecté, il est possible de substituer aux mesures perturbées des mesures obtenues à partir de l'appareil 50 ou de prendre en compte en plus des mesures obtenues à partir de l'appareil 50.

L'orientation de l'objet à localiser peut être définie par une autre méthode telle que des angles d'Euler ou des quaterrions.

Ce qui a été décrit ne s'applique pas uniquement au domaine médical mais au contraire à tout domaine où il est nécessaire de détecter un perturbateur ou de localiser un objet en utilisant des champs magnétiques.

## Revendications

1. Procédé de détection d'un perturbateur d'amplitude de champ magnétique à proximité d'une source monoaxe de ce champ magnétique, ce procédé comprenant les étapes suivantes:

   a) l'émission (62) d'au moins trois champs magnétiques $B_{11}$, $B_{12}$,... $B_{1j}$ de fréquences différentes $f_1$, $f_2$,... $f_j$ à partir d'une même source mono-axe de champ magnétique, les amplitudes des champs magnétiques émis à deux fréquences différentes quelconques étant reliées l'une à l'autre par un rapport prédéterminé,
   b) la mesure (64) de l'amplitude de ces champs magnétiques de fréquences différentes à l'aide d'un même capteur, et
   c) le signalement (70) d'un perturbateur d'amplitude si un rapport entre deux desdites amplitudes mesurées s'écarte d'un seuil prédéterminé du rapport prédéterminé qui relie les amplitudes des champs magnétiques émis, auxdites deux mêmes fréquences différentes d'émission, et, dans le cas contraire, l'absence de signalement

   **caractérisé en ce que** :

   - lors de l'étape a), les amplitudes des champs magnétiques $B_{11}$, $B_{12}$,... $B_{1j}$ émis sont toutes égales de sorte que les rapports entre les amplitudes des champs magnétiques émis aux différentes fréquences sont tous égaux à un, et
   - l'étape c) comporte la vérification que les rapports entre les amplitudes mesurées pour différentes fréquences sont constants quelle que soit la fréquence.

2. Procédé selon la revendication 1, dans lequel le procédé comprend :

   - la synchronisation de l'émission et de la mesure des champs magnétiques de fréquences différentes pour mesurer (62) uniquement l'amplitude du champ magnétique en phase avec le champ magnétique émis auxdites mêmes fréquences différentes, et
- le signalement (70) ou non d'un perturbateur d'amplitude uniquement en fonction des amplitudes mesurées des champs magnétiques en phase.

3. Procédé selon l'une quelconque des revendications précédentes, dans lequel les champs magnétiques de fréquences différentes sont émis simultanément.

4. Procédé de localisation d'un objet dans un référentiel à l'aide d'un détecteur comportant au moins une paire de mesures, chaque paire de mesures comportant :

- une source monoaxe apte à émettre des champs magnétiques à plusieurs fréquences, et
- un capteur pour mesurer l'amplitude des champs magnétiques émis par cette source monoaxe,
- l'un de la source monoaxe et du capteur étant solidaire du référentiel et l'autre de la source monoaxe et du capteur étant solidaire de l'objet,

ce procédé comportant :

- l'émission (82) d'un champ magnétique à une fréquence donnée par la source monoaxe et la mesure (84) de ce champ magnétique par le capteur de la même paire de mesures du détecteur,
- la localisation (86) de l'objet dans le référentiel à partir des mesures réalisées par la ou les paire(s) de mesures du détecteur,

**caractérisé en ce que** ce procédé comprend également :

- la détection (60 ; 88) d'un perturbateur d'amplitude du champ magnétique en utilisant la source monoaxe et le capteur de cette paire de mesures du détecteur pour mettre en œuvre un procédé conforme à l'une quelconque des revendications précédentes, et
- lors de la localisation de l'objet, la pondération de la mesure du champ magnétique réalisée par cette paire de mesures du détecteur en fonction du signalement ou non d'un perturbateur de manière à limiter l'impact d'une mesure perturbée réalisée par cette paire de mesures du détecteur, sur la localisation de l'objet.

5. Procédé selon la revendication 4, dans lequel le procédé comprend :

- l'émission (82) de champs magnétiques et la mesure (84) de ces champs magnétiques à l'aide de plus de neuf paires de mesures du détecteur, chaque paire de mesures se distinguant des autres par la source monoaxe ou le capteur utilisé ou par la fréquence du champ magnétique mesurée, et
- lors de la localisation (86) de l'objet, la mesure d'une paire de mesures du détecteur signalée comme perturbée par un perturbateur est pondérée de manière à limiter l'impact de cette mesure perturbée sur la localisation de l'objet par rapport aux mesures des paires de mesures du détecteur non perturbées.

6. Support (46) d'enregistrement d'informations, **caractérisé en ce qu'**il comporte des instructions pour la mise en œuvre d'un procédé conforme à l'une quelconque des revendications précédentes, lorsque ces instructions sont exécutées par un calculateur électronique.

7. Détecteur d'un perturbateur d'amplitude de champ magnétique, ce détecteur comprenant :

- une source monoaxe (32-34) d'au moins trois champs magnétiques $B_{11}$, $B_{12}$,... $B_{1j}$ de fréquences différentes $f_1$, $f_2$,... $f_j$ dans laquelle les amplitudes des champs magnétiques émis à deux fréquences différentes quelconques sont reliées l'une à l'autre par un rapport prédéterminé,
- un capteur (10) pour mesurer les amplitudes de ces champs magnétiques de fréquences différentes, et
- un indicateur (40) apte à signaler la présence d'un perturbateur de l'amplitude du champ magnétique seulement si un rapport entre deux desdites amplitudes mesurées s'écarte d'un seuil prédéterminé du rapport prédéterminé qui relie les amplitudes des champs magnétiques émis auxdites deux mêmes fréquences différentes d'émission,

**caractérisé en ce que** .:

- la source monoaxe (32-34) est configurée pour que les amplitudes des champs magnétiques $B_{11}$, $B_{12}$,... $B_{1j}$ émis soient toutes égales de sorte que les rapports entre les amplitudes des champs magnétiques émis aux différentes fréquences sont tous égaux à un, et

- l'indicateur est configuré pour vérifier que les rapports entre les amplitudes mesurées pour différentes fréquences sont constants quelle que soit la fréquence.

**8.** Détecteur selon la revendication 7, dans lequel la source monoaxe (32-34) est constituée d'une seule bobine dont les spires sont enroulées autour d'un seul axe d'enroulement.

**9.** Système de localisation d'un objet dans un référentiel, ce système comportant :

- au moins une paire de mesures, chaque paire de mesures étant formée par :

  • une source monoaxe (32-34) apte à émettre des champs magnétiques à plusieurs fréquences, et
  • un capteur (10) pour mesurer l'amplitude des champs magnétiques émis par cette source monoaxe,
  • l'un de la source monoaxe et du capteur étant solidaire du référentiel et l'autre de la source monoaxe et du capteur étant solidaire de l'objet,

- un module (42) de localisation apte à localiser l'objet dans le référentiel à partir des mesures réalisées par la ou les paire(s) de mesures,

**caractérisé en ce que** le système comprend :

- un détecteur (26, 27A, 27B, 10, 24, 40) d'un perturbateur d'amplitude de champ magnétique perturbant cette paire de mesures conforme à l'une quelconque des revendications 7 ou 8, la source monoaxe et le capteur de ce détecteur étant commun avec ceux de la paire de mesures, et
- le module (42) de localisation est apte à pondérer la mesure du champ magnétique réalisée par cette paire de mesures en fonction du signalement ou non d'un perturbateur de manière à limiter l'impact d'une mesure perturbée réalisée par cette paire de mesures, sur la localisation de l'objet.

**Patentansprüche**

**1.** Verfahren zur Detektion eines Magnetfeldamplituden-Störobjekts in der Nähe einer einachsigen Quelle dieses Magnetfeldes, wobei dieses Verfahren die folgenden Schritte umfasst:

a) das Aussenden (62) von wenigstens drei Magnetfeldern $B_{11}$, $B_{12}$, ..., $B_{1j}$ mit unterschiedlichen Frequenzen $f_1$, $f_2$, ..., $f_j$ von derselben einachsigen Magnetfeldquelle aus, wobei die Amplituden der mit beliebigen zwei verschiedenen Frequenzen ausgesendeten Magnetfelder durch ein vorbestimmtes Verhältnis miteinander verknüpft sind,

b) die Messung (64) der Amplitude dieser Magnetfelder mit verschiedenen Frequenzen mithilfe desselben Sensors, und

c) die Meldung (70) eines Amplitudenstörobjekts, falls ein Verhältnis zwischen zwei der gemessenen Amplituden um einen vorbestimmten Schwellenwert von dem vorbestimmten Verhältnis, welches die Amplituden der mit denselben zwei verschiedenen Sendefrequenzen ausgesendeten Magnetfelder verknüpft, abweicht, und andernfalls das Ausbleiben einer Meldung,

**dadurch gekennzeichnet, dass**:

- im Schritt a) die Amplituden der ausgesendeten Magnetfelder $B_{11}$, $B_{12}$, ..., $B_{1j}$ alle gleich sind, so dass die Verhältnisse zwischen den Amplituden der mit den unterschiedlichen Frequenzen ausgesendeten Magnetfelder alle gleich eins sind, und
- der Schritt c) die Überprüfung beinhaltet, ob die Verhältnisse zwischen den für verschiedene Frequenzen gemessenen Amplituden konstant sind, unabhängig von der Frequenz.

**2.** Verfahren nach Anspruch 1, wobei das Verfahren umfasst:

- die Synchronisation des Aussendens und der Messung der Magnetfelder mit unterschiedlichen Frequenzen, um ausschließlich die Amplitude des Magnetfeldes zu messen (62), das in Phase mit dem mit denselben unterschiedlichen Frequenzen ausgesendeten Magnetfeld ist, und

- die Meldung (70) oder Nichtmeldung eines Amplitudenstörobjekts ausschließlich in Abhängigkeit von den gemessenen Amplituden der in Phase befindlichen Magnetfelder.

3. Verfahren nach einem der vorhergehenden Ansprüche, wobei die Magnetfelder mit unterschiedlichen Frequenzen gleichzeitig ausgesendet werden.

4. Verfahren zur Positionsbestimmung eines Objekts in einem Koordinatensystem mithilfe eines Detektors, der wenigstens ein Messpaar aufweist, wobei jedes Messpaar aufweist:

   - eine einachsige Quelle, die in der Lage ist, Magnetfelder mit mehreren Frequenzen auszusenden, und
   - einen Sensor zum Messen der Amplitude der von dieser einachsigen Quelle ausgesendeten Magnetfelder,
   - wobei eines von der einachsigen Quelle und dem Sensor mit dem Koordinatensystem fest verbunden ist und das andere von der einachsigen Quelle und dem Sensor mit dem Objekt fest verbunden ist,

   wobei dieses Verfahren umfasst:

   - das Aussenden (82) eines Magnetfeldes mit einer gegebenen Frequenz durch die einachsige Quelle und die Messung (84) dieses Magnetfeldes durch den Sensor desselben Messpaares des Detektors,
   - die Positionsbestimmung (86) des Objekts in dem Koordinatensystem anhand der von dem oder den Messpaar(en) des Detektors durchgeführten Messungen, **dadurch gekennzeichnet, dass** das Verfahren außerdem umfasst:
   - die Detektion (60; 88) eines Amplitudenstörobjekts des Magnetfeldes unter Verwendung der einachsigen Quelle und des Sensors dieses Messpaares des Detektors, um ein Verfahren gemäß einem der vorhergehenden Ansprüche durchzuführen, und
   - bei der Positionsbestimmung des Objekts, die Gewichtung der von diesem Messpaar des Detektors durchgeführten Messung des Magnetfeldes in Abhängigkeit von der Meldung oder Nichtmeldung eines Störobjekts, um so die Auswirkung einer von diesem Messpaar des Detektors durchgeführten Messung auf die Positionsbestimmung des Objekts zu begrenzen.

5. Vorrichtung nach Anspruch 4, wobei das Verfahren umfasst:

   - das Aussenden (82) von Magnetfeldern und die Messung (84) dieser Magnetfelder mithilfe von mehr als neun Messpaaren des Detektors, wobei sich jedes Messpaar von den anderen durch die einachsige Quelle oder den verwendeten Sensor oder durch die gemessene Frequenz des Magnetfeldes unterscheidet, und
   - bei der Positionsbestimmung (86) des Objekts wird die Messung eines Messpaares des Detektors, die als durch ein Störobjekt gestört gemeldet wird, gewichtet, um so die Auswirkung dieser gestörten Messung auf die Positionsbestimmung des Objekts gegenüber den nicht gestörten Messungen der Messpaare des Detektors zu begrenzen.

6. Informationsaufzeichnungsmedium (46), **dadurch gekennzeichnet, dass** es Anweisungen zur Durchführung eines Verfahrens gemäß einem der vorhergehenden Ansprüche, wenn diese Anweisungen von einem elektronischen Rechner ausgeführt werden, umfasst.

7. Detektor eines Magnetfeldamplituden-Störobjekts, wobei dieser Detektor umfasst:

   - eine einachsige Quelle (32-34) von wenigstens drei Magnetfeldern $B_{11}$, $B_{12}$, ..., $B_{1j}$ mit unterschiedlichen Frequenzen $f_1$, $f_2$, ..., $f_j$, wobei die Amplituden der mit beliebigen zwei verschiedenen Frequenzen ausgesendeten Magnetfelder durch ein vorbestimmtes Verhältnis miteinander verknüpft sind,
   - einen Sensor (10) zum Messen der Amplituden dieser Magnetfelder mit verschiedenen Frequenzen, und
   - eine Anzeigevorrichtung (40), die in der Lage ist, das Vorhandensein eines Amplitudenstörobjekts des Magnetfeldes nur dann zu melden, falls ein Verhältnis zwischen zwei der gemessenen Amplituden um einen vorbestimmten Schwellenwert von dem vorbestimmten Verhältnis, welches die Amplituden der mit denselben zwei verschiedenen Sendefrequenzen ausgesendeten Magnetfelder verknüpft, abweicht,

   **dadurch gekennzeichnet, dass**:

   - die einachsige Quelle (32-34) derart ausgelegt ist, dass die Amplituden der ausgesendeten Magnetfelder $B_{11}$, $B_{12}$, ..., $B_{1j}$ alle gleich sind, so dass die Verhältnisse zwischen den Amplituden der mit den unterschiedlichen Frequenzen ausgesendeten Magnetfelder alle gleich eins sind, und
- die Anzeigevorrichtung dafür ausgelegt ist zu überprüfen, ob die Verhältnisse zwischen den für verschiedene Frequenzen gemessenen Amplituden konstant sind, unabhängig von der Frequenz.

8.  Detektor nach Anspruch 7, wobei die einachsige Quelle (32-34) aus einer einzigen Spule besteht, deren Windungen um eine einzige Wickelachse gewickelt sind.

9.  System zur Positionsbestimmung eines Objekts in einem Koordinatensystem, wobei dieses System aufweist:

    - wenigstens ein Messpaar, wobei jedes Messpaar gebildet wird von:

        • einer einachsigen Quelle (32-34), die in der Lage ist, Magnetfelder mit mehreren Frequenzen auszusenden, und
        • einem Sensor (10) zum Messen der Amplitude der von dieser einachsigen Quelle ausgesendeten Magnetfelder,
        • wobei eines von der einachsigen Quelle und dem Sensor mit dem Koordinatensystem fest verbunden ist und das andere von der einachsigen Quelle und dem Sensor mit dem Objekt fest verbunden ist,

    - ein Positionsbestimmungsmodul (42), das in der Lage ist, die Position des Objekts in dem Koordinatensystem anhand der von dem oder den Messpaar(en) durchgeführten Messungen zu bestimmen,

    **dadurch gekennzeichnet, dass** das System umfasst:

        - einen Detektor (26, 27A, 27B, 10, 24, 40) eines Magnetfeldamplituden-Störobjekts, das dieses Messpaar stört, gemäß einem der Ansprüche 7 oder 8, wobei die einachsige Quelle und der Sensor dieses Detektors dieselben sind wie diejenigen des Messpaares, und
        - das Positionsbestimmungsmodul (42) in der Lage ist, die von diesem Messpaar durchgeführte Messung des Magnetfeldes in Abhängigkeit von der Meldung oder Nichtmeldung eines Störobjekts zu gewichten, um so die Auswirkung einer von diesem Messpaar durchgeführten Messung auf die Positionsbestimmung des Objekts zu begrenzen.

**Claims**

1.  Method for detecting a disruptor of magnetic-field amplitude in proximity to a mono-axial magnetic-field source, this method comprising the following steps:

    a) emitting (62) at least three magnetic fields $B_{11}$, $B_{12}$... $B_{1j}$ of different frequencies $f_1$, $f_2$,... $f_j$ from the same mono-axial magnetic-field source, the amplitudes of the magnetic fields emitted at any two different frequencies being related to each other by a preset ratio,
    b) measuring (64) the amplitude of these magnetic fields of different frequencies using the same sensor, and
    c) signalling (70) an amplitude disruptor if a ratio between two of said measured amplitudes diverges from a preset threshold of the preset ratio that relates the amplitudes of the emitted magnetic fields, at said two different emission frequencies, and, in the contrary case, not signalling

    **characterized in that**:

    - in step a), the amplitudes of the emitted magnetic fields $B_{11}$, $B_{12}$... $B_{1j}$ are all equal so that the ratios between the amplitudes of the magnetic fields emitted at the various frequencies are all equal to one, and
    - step c) comprises verifying that the ratios between the amplitudes measured for various frequencies are constant whatever the frequency.

2.  Method according to Claim 1, wherein the method comprises:

    - synchronizing the emission and the measurement of the magnetic fields of different frequencies in order to measure (62) solely the amplitude of the magnetic field in phase with the magnetic field emitted at said given different frequencies, and
    - signalling (70) or not signalling an amplitude disruptor solely depending on the measured amplitudes of the in-phase magnetic fields.

3. Method according to any one of the preceding claims, wherein the magnetic fields of different frequencies are emitted simultaneously.

4. Method for locating an object in a frame of reference using a detector comprising at least one pair of measurements, each pair of measurements comprising:

- a mono-axial source able to emit magnetic fields at a plurality of frequencies, and
- a sensor for measuring the amplitude of the magnetic fields emitted by this mono-axial source,
- one of the mono-axial source and the sensor being tied to the frame of reference and the other of the mono-axial source and the sensor being fastened to the object,

this method comprising:

- emitting (82) a magnetic field at a given frequency using the mono-axial source and measuring (84) this magnetic field using the sensor of the given pair of measurements of the detector,
- locating (86) the object in the frame of reference on the basis of the measurements carried out by the one or more pairs of measurements of the detector,

**characterized in that** this method also comprises:

- detecting (60; 88) a disruptor of the amplitude of the magnetic field using the mono-axial source and the sensor of this pair of measurements of the detector to implement a method according to any one of the preceding claims, and
- while locating the object, weighting the measurement of the magnetic field carried out by this pair of measurements of the detector depending on whether or not a disruptor is signalled so as to limit the impact of a disrupted measurement carried out by this pair of measurements of the detector, on the location of the object.

5. Method according to Claim 4, wherein the method comprises:

- emitting (82) magnetic fields and measuring (84) these magnetic fields using more than nine pairs of measurements of the detector, each pair of measurements differing from the others in the mono-axial source or sensor used or in the magnetic-field frequency measured, and
- while locating (86) the object, the measurement of a pair of measurements of the detector signalled as disrupted by a disruptor is weighted so as to limit the impact of this disrupted measurement on the location of the object with respect to the measurements of the pairs of measurements of the detector that are not disrupted.

6. Data-storage medium (46), **characterized in that** it contains instructions for implementing a method according to any one of the preceding claims, when these instructions are executed by an electronic processor.

7. Detector of a disruptor of magnetic-field amplitude, this detector comprising:

- a mono-axial source (32-34) of at least three magnetic fields $B_{11}$, B12... $B_{1j}$ of different frequencies $f_1$, $f_2$,... $f_j$, in which source the amplitudes of the magnetic fields emitted at any two different frequencies are related to each other by a preset ratio,
- a sensor (10) for measuring the amplitudes of these magnetic fields of different frequencies, and
- an indicator (40) able to signal the presence of a disruptor of the amplitude of the magnetic field only if a ratio between two of said measured amplitudes diverges from a preset threshold of the preset ratio that relates the amplitudes of the emitted magnetic fields, at said two different emission frequencies, **characterized in that**:
- the mono-axial source (32-34) is configured so that the amplitudes of the emitted magnetic fields $B_{11}$, $B_{12}$... $B_{1j}$ are all equal so that the ratios between the amplitudes of the magnetic fields emitted at the various frequencies are all equal to one, and
- the indicator is configured to verify that the ratios between the amplitudes measured for various frequencies are constant whatever the frequency.

8. Detector according to Claim 7, wherein the mono-axial source (32-34) consists of a single coil the turns of which are wound around a single winding axis.

9. System for locating an object in a frame of reference, this system comprising:

- at least one pair of measurements, each pair of measurements comprising:

  • a mono-axial source (32-34) able to emit magnetic fields at a plurality of frequencies, and
  • a sensor (10) for measuring the amplitude of the magnetic fields emitted by this mono-axial source,
  • one of the mono-axial source and the sensor being tied to the frame of reference and the other of the mono-axial source and the sensor being fastened to the object,

- a locating module (42) able to locate the object in the frame of reference on the basis of the measurements carried out by the one or more pairs of measurements,

**characterized in that** the system comprises:

- a detector (26, 27A, 27B, 10, 24, 40) of a disruptor of the amplitude of the magnetic field disrupting this pair of measurements according to either one of Claims 7 and 8, the mono-axial source and the sensor of this detector being common to those of the pair of measurements, and
- the locating module (42) is able to weight the measurement of the magnetic field carried out by this pair of measurements depending on whether or not a disruptor is signalled so as to limit the impact of a disrupted measurement carried out by this pair of measurements of the detector, on the location of the object.

Fig. 1

EP 2 435 861 B1

Fig. 2

Fig. 4

Fig. 3

Fig. 5

## RÉFÉRENCES CITÉES DANS LA DESCRIPTION

**Documents brevets cités dans la description**

- EP 1502544 A
- EP 0993804 A
- US 2002092338 A
- EP 0319637 A
- EP 2052681 A
- US 6528989 B